Europäisches Patentamt

European Patent Office

Office européen des brevets

(19)

(11) Numéro de publication: **0 515 258 B1**

(12)

# FASCICULE DE BREVET EUROPEEN

(45) Date de publication de fascicule du brevet: **31.08.94**   (51) Int. Cl.⁵: **C07C 19/08**, C07C 17/20

(21) Numéro de dépôt: **92401350.1**

(22) Date de dépôt: **18.05.92**

(54) **Synthèse de bromures de perfluoroalkyle.**

(30) Priorité: **21.05.91 FR 9106114**

(43) Date de publication de la demande:
**25.11.92 Bulletin 92/48**

(45) Mention de la délivrance du brevet:
**31.08.94 Bulletin 94/35**

(84) Etats contractants désignés:
**AT BE CH DK ES GR LI LU NL PT SE**

(56) Documents cités:
**EP-A- 0 194 781**
**EP-A- 0 450 584**
**DE-A- 4 116 361**
**US-A- 5 072 062**

**CHEMICAL ABSTRACTS, vol. 104, no. 11, 17
Mars 1986, Columbus, Ohio, US; abstract no.
88106P, page 619 ;**

(73) Titulaire: **ELF ATOCHEM S.A.
4 & 8, Cours Michelet
La Défense 10
F-92800 Puteaux (FR)**

(72) Inventeur: **Drivon, Gilles
3 rue Joanny Courbière
F-69850 Saint-Martin-En Haut (FR)**

(74) Mandataire: **Leboulenger, Jean et al
Elf Atochem S.A.
Département Propriété Industrielle, Cedex 42
- La Défense 10
F-92091 Paris la Défense (FR)**

Rank Xerox (UK) Business Services
(3. 10/3.09/3.3.3)

## Description

La présente invention concerne le domaine des hydrocarbures aliphatiques perhalogénés et a plus particulièrement pour objet la préparation des bromures de perfluoroalkyle ou bromoperfluoroalcanes $R_F$-Br, $R_F$ désignant un radical perfluoroalkyle $C_nF_{2n+1}$, linéaire ou ramifié, contenant de 2 à 12 atomes de carbone.

Ces composés connus sont utilisés dans de nombreux domaines, en particulier en médecine comme radiopaques (agents de contraste aux rayons X) ou comme transporteurs d'oxygène dans les substituts du sang. Un composé plus spécialement étudié dans ce domaine est le bromure de n.perfluorooctyle $C_8F_{17}Br$.

Parmi les méthodes connues pour préparer ces composés, on peut d'abord signaler :
- l'action du brome sur un composé $R_FSF_5$ à 500°C en présence de nickel (brevet US 3 456 024) ;
- la photolyse en phase gazeuse d'un composé $R_FH$ par Br-Cl ou Br-F (J.L. Adcock et al, CA 100, 34092 e) ou par $Br_2$ (brevet FR 1 512 068).

La faiblesse des rendements obtenus et/ou l'utilisation de dérivés fluorés non disponibles industriellement ne permettent pas une production économique des composés $R_FBr$ à l'échelle industrielle.

Dans son brevet EP 0 298 870 et sa demande de brevet EP 90403118.4, la Demanderesse a décrit des procédés de fabrication des composés $R_FBr$ à partir des chlorures de perfluoroalcane-sulfonyle $R_FSO_2Cl$ correspondants qu'on fait réagir soit avec HBr gazeux en présence d'un catalyseur (EP 0 298 870), soit avec un bromure d'ammonium ou de phosphonium quaternaire (EP 90403118.4). Les rendements obtenus sont généralement élevés, mais le sulfochlorure $R_FSO_2Cl$ utilisé est une matière première déjà très élaborée puisque sa synthèse à partir de l'iodure correspondant $R_FI$ nécessite deux étapes réactionnelles suivant l'équation :

$$2 \ R_FI + 2 \ SO_2 \ \xrightarrow{\ Zn\ } \ (R_FSO_2)_2Zn \ \xrightarrow{\ 2Cl_2\ } \ 2 \ R_FSO_2Cl + ZnCl_2$$

La voie la plus directe d'obtention des composés $R_FBr$ consiste évidemment en une bromation radicalaire des iodures correspondants $R_FI$, ces derniers étant des produits disponibles en quantités industrielles.

Dans International Journal of Chemical Kinetics, vol. II, 273-285 (1975), E.N. Okafo et E. Whittle décrivent la cinétique de bromation thermique de $CF_3I$ dans un réacteur photochimique entre 173 et 321°C, en vue de déterminer l'énergie de dissociation de la liaison C-I.

Dans J. Chem. Soc. 1953, 3761-8, R.N. Haszeldine a décrit un procédé photochimique de réaction des $R_FI$ avec du brome en opérant en tube scellé avec un excès de brome (10 %) et en irradiant par une lumière UV pendant sept jours. La température réactionnelle et la pureté obtenue ne sont pas précisées ; il est simplement indiqué que le rendement est supérieur ou égal à 90 % selon la longueur de la chaîne perfluorée $R_F$.

Dans les exemples de la demande japonaise Kokai 85-184033 qui décrit la réaction des $R_FI$ avec $Br_2$ en présence d'un initiateur chimique de radicaux, les rendements indiqués ne dépassent pas 42 %.

La faiblesse des rendements obtenus et/ou la cinétique lente de ces techniques ne permettent pas d'envisager leur exploitation industrielle.

Il a maintenant été trouvé un procédé permettant la production continue des bromures de perfluoroalkyle $R_FBr$ contenant de 2 à 12 atomes de carbone, de préférence 6 à 10, à partir des $R_FI$ correspondants, avec une sélectivité quasi-totale en $R_FBr$. Le procédé selon l'invention est caractérisé en ce que l'on fait réagir en continu un $R_FI$ et du brome en phase gazeuse.

La réaction peut être effectuée dans un réacteur tubulaire à une température allant de 200 à 600°C, mais on opère avantageusement à une température comprise entre 300 et 500°C, de préférence entre 350 et 500°C. Le réacteur peut être un tube vide, mais il peut éventuellement contenir un substrat solide inerte (par exemple verre ou quartz) pour faciliter le contact entre les deux gaz ($Br_2$ et $R_FI$). Bien que cela ne soit pas indispensable, on peut aussi opérer en présence d'un diluant gazeux inerte, par exemple l'azote.

La réaction du brome avec le $R_FI$ en phase gazeuse étant très rapide, le temps de contact, c'est-à-dire le temps de séjour des réactifs dans le réacteur, n'est pas un paramètre critique et peut varier dans de larges limites. Un temps de contact compris entre 1 seconde et 2 minutes convient généralement bien, mais industriellement on préfère opérer avec un temps de contact allant de 5 à 30 secondes.

Industriellement, on préfère travailler à la pression atmosphérique, mais on ne sortirait pas du cadre de la présente invention en travaillant à une pression supérieure à la pression atmosphérique, pourvu que le système réactionnel reste à l'état gazeux.

Pour récupérer le $R_FBr$ formé et éventuellement le $R_FI$ non transformé, les gaz sortant du réacteur peuvent être refroidis, puis le mélange est distillé directement ou après traitement par un agent réducteur.

Le procédé selon l'invention peut être mis en oeuvre en opérant avec un excès ou un défaut de brome par rapport à la quantité théorique (0,5 mole de $Br_2$ par mole de $R_FI$). Le rapport molaire $Br_2/R_FI$ peut donc varier dans de larges limites, notamment entre 0,1 et 2. Cependant, pour l'économie du procédé, on opère avantageusement à un rapport molaire $Br_2/R_FI$ compris entre 0,3 et 1.

Lorsqu'on opère avec un excès de brome et quels que soient la température et le temps de contact, la réaction se déroule selon le schéma :

$$R_FI + Br_2 \rightarrow R_FBr + BrI \qquad (1)$$

Suivant les conditions opératoires mises en oeuvre, il est alors possible d'obtenir une conversion quasi-complète du $R_FI$, ce qui facilite la récupération du $R_FBr$ à l'état très pur par simple distillation. Toutefois, du fait de l'équilibre thermodynamique :

$$BrI \rightleftharpoons \tfrac{1}{2} Br_2 + \tfrac{1}{2} I_2$$

on se retrouve obligatoirement en sortie de réacteur avec un mélange contenant du brome et de l'iode.

Lorsqu'on opère avec un défaut de brome et suivant les conditions opératoires (rapport molaire, température, temps de contact), la réaction se déroule soit selon le schéma (1) ci-dessus, soit selon le schéma (2) :

$$2\,R_FI + Br_2 \rightarrow 2\,R_FBr + I_2 \qquad (2)$$

Compte-tenu du défaut de brome, on n'obtient évidemment qu'une conversion partielle du $R_FI$. Par contre, on peut réunir des conditions opératoires permettant une conversion quasi-complète du brome (équation 2), ce qui simplifie la récupération et la valorisation de l'iode sous-produit sous sa forme oxydée ou réduite. Après séparation de l'iode, on peut distiller le mélange $R_FBr/R_FI$ pour séparer le $R_FI$ non converti et le recycler au réacteur. On peut aussi ajouter au mélange $R_FBr/R_FI$ un appoint de brome et terminer la réaction dans un second réacteur.

Le brome et le $R_FI$ peuvent être introduits séparément au réacteur. Cependant, le brome étant partiellement soluble dans le $R_FI$, on peut avantageusement, lorsqu'on travaille à un rapport molaire inférieur à la limite de solubilité, introduire les deux réactifs à partir d'un mélange homogène de ces deux composés ; ceci permet alors d'opérer avec une seule pompe d'alimentation et d'assurer ainsi un rapport molaire constant.

Le procédé selon l'invention s'applique aussi bien à la préparation d'un $R_FBr$ spécifique (par exemple $C_6F_{13}Br$, $C_8F_{17}Br$, $C_{10}F_{21}Br$, ...) qu'à celle d'un mélange de différents $R_FBr$ à partir d'un mélange des $R_FI$ correspondants.

Dans les exemples suivants qui illustrent l'invention sans la limiter, le taux de transformation (TT) du $R_FI$ est calculé par la relation :

$$TT = 100 \times \frac{\text{moles } R_FI \text{ entrée} - \text{moles } R_FI \text{ sortie}}{\text{moles } R_FI \text{ entrée}}$$

et la sélectivité (S) en $R_FBr$ par la relation :

S = 100 - (% de divers sortie - % d'impuretés entrée)

## EXEMPLE 1 : SYNTHESE DU BROMURE DE PERFLUOROOCTYLE

On utilise un réacteur tubulaire en verre (diamètre intérieur : 30 mm ; hauteur : 300 mm) garni au 1/5$^e$ par des anneaux pour assurer un bon mélange des gaz et équipé d'une gaine thermométrique pour le contrôle de la température et d'une canne plongeante pour l'introduction des réactifs.

Essai n° 1

A l'aide de deux pompes doseuses, on alimente ce réacteur simultanément et en continu pendant 2 heures avec 0,261 mole de brome et 0,560 mole de iodure de perfluorooctyle (pureté : 99,3 %), ainsi que 2 l/h d'azote. Les conditions opératoires sont les suivantes :
- température = 300°C
- temps de contact ≈ 30 secondes
- rapport molaire $Br_2/C_8F_{17}I$ = 0,47

Les gaz sortant du réacteur sont recueillis dans une solution aqueuse de sulfite de sodium en excès. Après décantation, on obtient deux phases :
- une phase aqueuse supérieure qui est dosée par argentimétrie pour déterminer la teneur en bromure et iodure présents ($I^-$ = 0,374 éq. et $Br^-$ = 0,150 éq.) ;
- une phase organique inférieure qui pèse 283,5 g et dont l'analyse par chromatographie en phase gazeuse sur un appareil VARIAN 3300 (détecteur catharomètre ; colonne DB1 macrobore 30 m) donne la composition pondérale suivante :

    $C_8F_{17}Br$ = 62,55 %
    $C_8F_{17}I$ = 36,80 %
    divers = 0,65 %

ce qui correspond à un taux de transformation du $C_8F_{17}I$ égal à 66 % et à une sélectivité en $C_8F_{17}Br$ de 100 %.

Essais n° 2 à 26

Ces essais, effectués dans le même appareillage et en opérant de la même façon que pour l'essai n° 1, montrent l'influence des différents paramètres sur le taux de transformation et la sélectivité.

Les conditions opératoires, les quantités mises en oeuvre et les résultats obtenus dans ces essais sont résumés dans les tableaux I à V suivants.

EP 0 515 258 B1

## TABLEAU I

### INFLUENCE DE LA TEMPERATURE DE REACTION

Temps de contact : 30 ± 2 secondes
Rapport molaire $Br_2/C_8F_{17}I$ : 1 ± 0,1
Pureté du $C_8F_{17}I$ de départ : 99,3 %
Durée d'un essai : environ 2 heures

| ESSAI N° | Conditions opératoires | | Quantités mises en oeuvre | | SORTIE REACTEUR | | | | | | | |
|---|---|---|---|---|---|---|---|---|---|---|---|---|
| | | | | | Phase organique | | | | | | Phase aqueuse | |
| | Température °C | Rap.mol. $Br_2/C_8F_{17}I$ | $Br_2$ mole | $C_8F_{17}I$ mole | Poids (g) | $C_8F_{17}Br$ mole | $C_8F_{17}I$ mole | TT % | Divers % | S % | $I^-$ eq. | $Br^-$ eq. |
| 2 | 250 | 1,07 | 0,423 | 0,396 | 200,8 | 0,153 | 0,228 | 42,5 | 0,64 | 100 | 0,155 | 0,716 |
| 3 | 300 | 0,92 | 0,340 | 0,368 | 179,2 | 0,294 | 0,058 | 84 | 0,70 | 100 | 0,312 | 0,419 |
| 4 | 350 | 0,99 | 0,342 | 0,344 | 167,6 | 0,334 | 0,001 | 99,7 | 0,66 | 100 | 0,356 | 0,357 |
| 5 | 400 | 0,94 | 0,303 | 0,322 | 156,6 | 0,314 | ε | #100 | 0,66 | 100 | 0,336 | 0,312 |
| 6 | 450 | 0,91 | 0,269 | 0,297 | 145,3 | 0,290 | ε | #100 | 0,72 | 100 | 0,312 | 0,266 |
| 7 | 500 | 1,09 | 0,300 | 0,276 | 133,4 | 0,264 | ε | #100 | 1,42 | 99,3 | 0,287 | 0,343 |

## TABLEAU II

### INFLUENCE DU RAPPORT MOLAIRE $Br_2/C_8F_{17}I$ A 300°C

Temps de contact : 30 ± 2 secondes
Pureté du $C_8F_{17}I$ de départ : 99,3 %
Durée d'un essai : environ 2 heures

| ESSAI N° | Rapport molaire $Br_2/C_8F_{17}I$ | Quantités mises en oeuvre | | SORTIE REACTEUR | | | | | | Phase aqueuse | |
|---|---|---|---|---|---|---|---|---|---|---|---|
| | | | | Phase organique | | | | | | | |
| | | $Br_2$ mole | $C_8F_{17}I$ mole | Poids (g) | $C_8F_{17}Br$ mole | $C_8F_{17}I$ mole | TT % | Divers % | S % | $I^-$ eq. | $Br^-$ eq. |
| 8 | 0,30 | 0,200 | 0,669 | 344,5 | 0,311 | 0,344 | 48,5 | 0,70 | 100 | 0,316 | 0,072 |
| 9 | 0,32 | 0,199 | 0,621 | 319,2 | 0,315 | 0,293 | 53 | 0,8 | 99,9 | 0,328 | 0,060 |
| 10 | 0,36 | 0,225 | 0,621 | 315,4 | 0,345 | 0,256 | 59 | 0,95 | 99,75 | 0,354 | 0,090 |
| 1 | 0,47 | 0,261 | 0,560 | 283,5 | 0,356 | 0,191 | 66 | 0,65 | 100 | 0,374 | 0,150 |
| 11 | 0,52 | 0,326 | 0,621 | 313,4 | 0,420 | 0,187 | 70 | 0,70 | 100 | 0,432 | 0,208 |
| 3 | 0,92 | 0,340 | 0,368 | 179,2 | 0,294 | 0,058 | 84 | 0,70 | 100 | 0,312 | 0,419 |

EP 0 515 258 B1

EP 0 515 258 B1

## TABLEAU III

### INFLUENCE DU RAPPORT MOLAIRE $Br_2/C_8F_{17}I$ A 350°C

Temps de contact : 30 ± 2 secondes
Pureté du $C_8F_{17}I$ de départ : 99,3 %
Durée d'un essai : environ 2 heures

| ESSAI N° | Rapport molaire $Br_2/C_8F_{17}I$ | Quantités mises en oeuvre | | SORTIE REACTEUR | | | | | | | |
|---|---|---|---|---|---|---|---|---|---|---|---|
| | | | | Phase organique | | | | | | Phase aqueuse | |
| | | $Br_2$ mole | $C_8F_{17}I$ mole | Poids (g) | $C_8F_{17}Br$ mole | $C_8F_{17}I$ mole | TT % | Divers % | S % | $I^-$ eq. | $Br^-$ eq. |
| 12 | 0,30 | 0,182 | 0,606 | 310,7 | 0,341 | 0,255 | 58 | 0,4 | 100 | 0,334 | ε |
| 13 | 0,385 | 0,216 | 0,562 | 271,8 | 0,405 | 0,123 | 78 | 0,9 | 99,8 | 0,404 | 0,014 |
| 14 | 0,54 | 0,279 | 0,513 | 251,8 | 0,471 | 0,028 | 94,5 | 0,6 | 100 | 0,494 | 0,066 |
| 4 | 0,99 | 0,342 | 0,344 | 167,6 | 0,334 | 0,001 | 99,7 | 0,66 | 100 | 0,356 | 0,357 |

## TABLEAU IV

### INFLUENCE DU RAPPORT MOLAIRE $Br_2/C_8F_{17}I$ A 400°C

Temps de contact : 30 ± 2 secondes
Pureté du $C_8F_{17}I$ de départ : 99,3 %
Durée d'un essai : environ 2 heures

| ESSAI N° | Rapport molaire $Br_2/C_8F_{17}I$ | Quantités mises en oeuvre | | SORTIE REACTEUR | | | | | | Phase aqueuse | |
| --- | --- | --- | --- | --- | --- | --- | --- | --- | --- | --- | --- |
| | | | | Phase organique | | | | | | | |
| | | $Br_2$ mole | $C_8F_{17}I$ mole | Poids (g) | $C_8F_{17}Br$ mole | $C_8F_{17}I$ mole | TT % | Divers % | S % | $I^-$ eq. | $Br^-$ eq. |
| 15 | 0,30 | 0,161 | 0,533 | 283,8 | 0,300 | 0,231 | 56,5 | 0,60 | 100 | 0,304 | ε |
| 16 | 0,36 | 0,171 | 0,478 | 236,7 | 0,337 | 0,123 | 74 | 0,70 | 100 | 0,348 | ε |
| 17 | 0,37 | 0,178 | 0,478 | 240,2 | 0,355 | 0,112 | 76,5 | 0,90 | 99,8 | 0,364 | ε |
| 18 | 0,52 | 0,250 | 0,478 | 234,1 | 0,447 | 0,017 | 96,5 | 0,70 | 100 | 0,478 | 0,040 |
| 19 | 0,52 | 0,248 | 0,478 | 237,1 | 0,454 | 0,016 | 96,5 | 0,70 | 100 | 0,486 | 0,032 |
| 20 | 0,59 | 0,285 | 0,478 | 232,5 | 0,463 | ε | 100 | 0,60 | 100 | 0,491 | 0,087 |
| 5 | 0,94 | 0,303 | 0,322 | 156,6 | 0,314 | ε | 100 | 0,66 | 100 | 0,336 | 0,312 |

EP 0 515 258 B1

EXEMPLE 2 : SYNTHESE DU BROMURE DE PERFLUOROHEXYLE

## TABLEAU V

Temps de contact : 10 ± 1 secondes
Pureté du $C_8F_{17}I$ de départ : 99,1 %
Durée d'un essai : environ 1 heure
Débit d'azote : 2,2 à 4,3 l/h

| ESSAI N° | Conditions opératoires | | Quantités mises en oeuvre | | SORTIE REACTEUR | | | | | | | |
|---|---|---|---|---|---|---|---|---|---|---|---|---|
| | | | | | Phase organique | | | | | | Phase aqueuse | |
| | Tempé-rature °C | Rap.mol. $Br_2/$ $C_8F_{17}I$ | $Br_2$ mole | $C_8F_{17}I$ mole | Poids (g) | $C_8F_{17}Br$ mole | $C_8F_{17}I$ mole | TT % | Divers % | S % | $I^-$ eq. | $Br^-$eq. |
| 21 | 300 | 0,30 | 0,329 | 1,100 | 563,1 | 0,511 | 0,554 | 49,5 | 1,0 | 99,9 | 0,518 | 0,114 |
| 22 | 350 | 0,30 | 0,300 | 0,996 | 513,6 | 0,569 | 0,412 | 58,5 | 0,9 | 100 | 0,564 | 0,018 |
| 23 | 400 | 0,30 | 0,278 | 0,927 | 475,5 | 0,553 | 0,356 | 61,5 | 1,0 | 99,9 | 0,536 | ε |
| 24 | 300 | 0,98 | 0,660 | 0,670 | 330 | 0,562 | 0,085 | 87,5 | 0,9 | 100 | 0,560 | 0,750 |
| 25 | 350 | 0,89 | 0,539 | 0,608 | 293,2 | 0,574 | 0,008 | 98,5 | 0,9 | 100 | 0,605 | 0,430 |
| 26 | 400 | 0,84 | 0,469 | 0,558 | 270,8 | 0,530 | 0,007 | 98,5 | 0,9 | 100 | 0,565 | 0,350 |

Essai n° 27

Dans le même appareillage qu'à l'exemple 1, on introduit en 40 minutes, simultanément et de façon continue 71 g (0,444 mole) de brome et 180 g (0,404 mole) de iodure de perfluorohexyle (pureté : 99,5 %) en présence de 8,4 l/h d'azote, dans les conditions opératoires suivantes :
- température : 350°C
- temps de contact : 8,6 secondes
- rapport molaire $Br_2/C_6F_{13}I$ : 1,1

En sortie du réacteur, après neutralisation dans une solution aqueuse de sulfite de sodium et décantation, on récupère une phase organique contenant 98,8 % en poids de $C_6F_{13}Br$ et 0,65 % de $C_6F_{13}I$, ce qui correspond à une sélectivité en $C_6F_{13}Br$ voisine de 100 %.

Essai n° 28

On part d'un mélange homogène de 441,7 g (0,99 mole) de $C_6F_{13}I$ et de 67,2 g (0,42 mole) de brome qu'on introduit en 168 minutes à l'aide d'une seule pompe doseuse dans le même réacteur qu'à l'exemple 1, en l'absence d'azote dans les conditions opératoires suivantes :
- température : 350°C
- temps de contact : 28 secondes
- rapport molaire $Br_2/C_6F_{13}I$ : 0,42

Après réduction au sulfite et décantation, on récupère en sortie de réacteur une phase organique contenant en poids 77,7 % de $C_6F_{13}Br$ et 21,8 % de $C_6F_{13}I$, ce qui correspond à une sélectivité en $C_6F_{13}Br$ de 100 %.

## Revendications

1. Procédé continu de préparation d'un bromure de perfluoroalkyle $R_F$-Br où $R_F$ désigne un radical perfluoroalkyle, linéaire ou ramifié, contenant de 2 à 12 atomes de carbone, par réaction du brome avec l'iodure de perfluoroalkyle $R_F$-I correspondant, caractérisé en ce qu'on effectue la réaction en phase gazeuse.

2. Procédé selon la revendication 1, dans lequel on opère à une température comprise entre 200 et 600°C, de préférence entre 350 et 500°C.

3. Procédé selon la revendication 1 ou 2, dans lequel le rapport molaire $Br_2/R_FI$ est compris entre 0,1 et 2, de préférence entre 0,3 et 1.

4. Procédé selon l'une des revendications 1 à 3, dans lequel on opère en présence d'un diluant inerte, de préférence l'azote.

5. Procédé selon l'une des revendications 1 à 4, dans lequel le temps de contact est compris entre 1 seconde et 2 minutes, de préférence entre 5 et 30 secondes.

6. Procédé selon l'une des revendications 1 à 5, dans lequel on opère à la pression atmosphérique.

7. Procédé selon l'une des revendications 1 à 6, dans lequel on introduit au réacteur un mélange homogène de brome et de $R_FI$.

8. Procédé selon l'une des revendications 1 à 7, dans lequel on opère avec un rapport molaire $Br_2/R_FI$ inférieur à 0,5, puis on sépare l'iode du mélange réactionnel pour obtenir un mélange $R_FBr/R_FI$ qu'on distille pour recycler le $R_FI$ ou auquel on ajoute un appoint de brome pour terminer la réaction dans un second réacteur.

9. Application du procédé selon l'une des revendications 1 à 8 à la synthèse du bromure de perfluorooctyle.

10. Application du procédé selon l'une des revendications 1 à 8 à la synthèse du bromure de perfluorohexyle.

**11.** Application du procédé selon l'une des revendications 1 à 8 à la synthèse du bromure de perfluorodécyle.

**12.** Application du procédé selon l'une des revendications 1 à 8 à la synthèse d'un mélange de bromures de perfluoroalkyle.

**Claims**

1. Continuous process for the preparation of a perfluoroalkyl bromide $R_F$-Br where $R_F$ denotes a linear or branched perfluoroalkyl radical containing from 2 to 12 carbon atoms, by reaction of bromine with the corresponding perfluoroalkyl iodide $R_F$-I, characterised in that the reaction is carried out in gaseous phase.

2. Process according to Claim 1, in which the operation is carried out at a temperature of between 200 and 600°C, preferably between 350 and 500°C.

3. Process according to Claim 1 or 2, in which the $Br_2/R_F I$ molar ratio is between 0.1 and 2, preferably between 0.3 and 1.

4. Process according to one of Claims 1 to 3, in which the operation is carried out in the presence of an inert diluent, preferably nitrogen.

5. Process according to one of Claims 1 to 4, in which the contact time is between 1 second and 2 minutes, preferably between 5 and 30 seconds.

6. Process according to one of Claims 1 to 5, in which the operation is carried out at atmospheric pressure.

7. Process according to one of Claims 1 to 6, in which a homogeneous mixture of bromine and $R_F I$ is introduced into the reactor.

8. Process according to one of Claims 1 to 7, in which the operation is carried out with a $Br_2/R_F I$ molar ratio lower than 0.5, and the iodine is then separated from the reaction mixture to obtain a $R_F Br/R_F I$ mixture which is distilled to recycle the $R_F I$ or to which make-up bromine is added to complete the reaction in a second reactor.

9. Application of the process according to one of Claims 1 to 8 to the synthesis of perfluorooctyl bromide.

10. Application of the process according to one of Claims 1 to 8 to the synthesis of perfluorohexyl bromide.

11. Application of the process according to one of Claims 1 to 8 to the synthesis of perfluorodecyl bromide.

12. Application of the process according to one of Claims 1 to 8 to the synthesis of a mixture of perfluoroalkyl bromides.

**Patentansprüche**

1. Kontinuierliches Verfahren zur Herstellung eines Perfluoralkylbromids $R_F$-Br, worin $R_F$ für einen linearen oder verzweigten Perfluoralkylrest mit 2 bis 12 C-Atomen steht, durch Umsetzung von Brom mit dem entsprechenden Perfluoralkyljodid $R_F$-I, dadurch gekennzeichnet, daß die Umsetzung in der Gasphase erfolgt.

2. Verfahren nach Anspruch 1, worin bei einer Temperatur zwischen 200 und 600°C, vorzugsweise zwischen 350 und 500°C gearbeitet wird.

3. Verfahren nach Anspruch 1 oder 2, bei dem das Molverhältnis $Br_2/R_F I$ zwischen 0,1 und 2, vorzugsweise zwischen 0,3 und 1 liegt.

4. Verfahren nach einem der Ansprüche 1 bis 3, bei dem in Gegenwart eines inerten Verdünnungsmittels, vorzugsweise Stickstoff, gearbeitet wird.

5. Verfahren anch einem der Ansprüche 1 bis 4, bei dem die Kontaktzeit zwischen 1 Sekunde und 2 Minuten, vorzugsweise zwischen 5 und 30 Sekunden, liegt.

6. Verfahren nach einem der Ansprüche 1 bis 5, bei dem bei atmosphärischem Druck gearbeitet wird.

7. Verfahren nach einem der Ansprüche 1 bis 6, bei dem in den Reaktor eine homogene Mischung aus Brom und $R_FI$ eingebracht wird.

8. Verfahren nach einem der Ansprüche 1 bis 7, bei dem mit einem Molverhältnis $Br_2/R_FI$ unter 0,5 gearbeitet, dann Jod von der Reaktionsmischung abgetrennt wird, um eine $R_FBr/R_FI$-Mischung zu erhalten, die destilliert wird, um das $R_FI$ rückzuführen oder dem man einen Bromzusatz zugibt, um die Reaktion in einem zweiten Reaktor abzuschließen.

9. Anwendung des Verfahrens nach einem der Ansprüche 1 bis 8 auf die Synthese von Perfluoroctylbromid.

10. Anwendung des Verfahrens nach einem der Ansprüche 1 bis 8 auf die Synthese von Perfluorhexylbromid.

11. Anwendung des Verfahrens nach einem der Ansprüche 1 bis 8 auf die Synthese von Perfluordecylbromid.

12. Anwendung des Verfahren nach einem der Ansprüche 1 bis 8 auf die Synthese einer Mischung aus Perfluoralkylbromiden.